**Europäisches Patentamt**

**European Patent Office**

**Office européen des brevets**

(11) Veröffentlichungsnummer: **0 240 855**
**A1**

## (12) EUROPÄISCHE PATENTANMELDUNG

(21) Anmeldenummer: **87104472.3**

(22) Anmeldetag: **26.03.87**

(51) Int. Cl.⁴: **C07C 85/11**

(30) Priorität: **08.04.86 DE 3611677**

(43) Veröffentlichungstag der Anmeldung:
**14.10.87 Patentblatt 87/42**

(84) Benannte Vertragsstaaten:
**BE DE ES FR GB IT NL**

(71) Anmelder: **BAYER AG**
**Konzernverwaltung RP Patentabteilung**
**D-5090 Leverkusen 1 Bayerwerk(DE)**

(72) Erfinder: **Beckhaus, Heiko, Dr.**
**Schulstrasse 20**
**D-2212 Brunsbüttel(DE)**
Erfinder: **Waldau, Eckart, Dr.**
**Benrather-Schlossufer 7**
**D-4000 Düsseldorf 13(DE)**
Erfinder: **Witt, Harro, Dr.**
**Möhlenbarg 2**
**D-2224 Kuden(DE)**

(54) Verfahren zur Herstellung von aromatischen Diaminen.

(57) Ein neues Verfahren zur Herstellung von aromatischen Diaminen durch katalytische Hydrierung der ihnen entsprechenden aromatischen Dinitroverbindungen in Gegenwart eines organischen Lösungsmittels, wobei man als organisches Lösungsmittel Tetrahydrofuran verwendet.

EP 0 240 855 A1

## Verfahren zur Herstellung von aromatischen Diaminen

Die Erfindung betrifft ein neues Verfahren zur Herstellung von aromatischen Diaminen durch Hydrierung von aromatischen Dinitroverbindungen unter Verwendung von Tetrahydrofuran als Lösungsmittel.

Es ist bekannt (vgl. DE-OSen 1 542 544, 1 947 851, 2 106 644, 2 135 154, 2 214 056, 2 456 308, 3 315 191, BE-PSen 631 964, 661 047, 661 946, FR-PS 1 359 438, GB-PS 768 111 oder EP-A-124 010), daß man aromatische Diamine durch katalytische Hydrierung von den entsprechenden aromatischen Dinitroverbindungen herstellen kann. Die Hydrierung kann unter Mitverwendung von Lösungsmitteln wie beispielsweise niedrigsiedenden Alkoholen wie Methanol, Ethanol oder Isopropanol durchgeführt werden; sie kann aber auch ohne solche Fremdlösungsmittel in der Mischung der Reaktionsprodukte erfolgen.

Die bislang in erster Linie eingesetzten Lösungsmittel (niedrigsiedende Alkohole) stehen unter den Hydrierbedingungen in einem Redoxgleichgewicht mit den entsprechenden Aldehyden bzw. Ketonen (vgl. V.N. Ipatieff et al., Ind. Eng. Chem. 41, 1802-1805 (1949)). Diese reagieren ihrerseits mit den aromatischen Diaminoverbindungen oder mit Ammoniak, das stets wegen hydrogenolytischer Abspaltungen von Aminogruppen aus dem Diamin im Reaktionsgemisch vorhanden ist, zu Azomethinen. Die Azomethine werden irreversibel zu den entsprechenden N-Alkylaminen hydriert und führen zu Ausbeuteminderung oder zu stark basischen, aliphatischen Aminen, im Falle des Isopropanols z.B. zum Isopropylamin. Diese stark basischen, aliphatischen Amine sind Kontaktgifte für die eingesetzten Katalysatoren vom Raney-Nickel-Typ und führen zu einer erhöhten Bildung von harzartigen Verbindungen auf Kosten der Ausbeute. Die harzartigen Verbindungen haben den weiteren Nachteil, daß sie die Wärmeaustauschflächen des Reaktors belegen und so zu einem schlechteren Wärmeübergang führen.

Alle diese unerwünschten Begleiterscheinungen verstärken sich mit steigenden Temperaturen, die ja gerade für eine optimale Energiegewinnung nützlich sind. Um diese unerwünschten Begleiteffekte soweit wie möglich auszuschließen, wurde bislang die großtechnische Hydrierung von aromatischen Dinitroverbindungen bei möglichst solchen niedrigen Temperaturen durchgeführt, was selbstverständlich einer Nutzung der Reaktionsenthalpie zur Erzeugung von höhergespanntem Dampf eines Druck von ca. 3 bis 20 bar abs. entgegensteht.

Es was daher die der Erfindung zugrundeliegende Aufgabe ein neues Verfahren zur Herstellung von aromatischen Diaminen zur Verfügung zu stellen, bie welchem ein Lösungsmittel eingesetzt wird, welches höhere Reaktionstemperaturen ohne erhöhte Nebenproduktbildung und damit die Erzeugung von höhergespanntem Dampf von ca. 3 bis 20 bar abs. gestattet, und dessen Mitverwendung eine höhere Produktausbeute als ein Arbeiten ohne Lösungsmittel ermöglicht.

Diese Aufgabe konnte durch die erfindungsgemäße Verwendung vom Tetrahydrofuran als Lösungsmittel für die beim erfindungsgemäßen Verfahren einzusetzenden Dinitroverbindungen bzw. die beim erfindungsgemäßen Verfahren resultierenden Diamine bzw. als Suspendiermittel für die beim erfindungsgemäßen Verfahren einzusetzenden Katalysatoren gelöst werden.

Gegenstand der Erfindung ist somit ein Verfahren zur Herstellung von aromatischen Diaminen durch katalytische Hydrierung der ihnen entsprechenden aromatischen Dinitroverbindungen in Gegenwart eines organischen Lösungsmittels, dadurch gekennzeichnet, daß man als organisches Lösungsmittel Tetrahydrofuran verwendet.

Beim erfindungsgemäßen Verfahren können beliebige aromatische Dinitroverbindungen eingesetzt werden. Beispiele geeigneter Dinitroverbindungen sind 1,3-Dinitrobenzol, 2,4-Dinitrotoluol, 2,6-Dinitrotoluol, im wesentlichen aus den beiden letztgenannten Isomeren bestehende, technische Dinitrotoluol-Gemische oder 1,5-Dinitronaphthalin. Besonders bevorzugt werden als aromatische Dinitroverbindungen 2,4-Dinitrotoluol oder dessen technische Gemische mit bis zu 35 Gew.-%, bezogen auf das Gesamtgemisch, an 2,6-Dinitrotoluol eingesetzt. Diese technische Gemische können auch untergeordnete Mengen, d.h. insgesamt bis maximal 5 Gew. -% bezogen auf Gesamtgemisch, an 2,3-, 2,5-und/oder 3,4-Dinitrotoluol enthalten.

Für das erfindungsgemäße Verfahren werden die an sich bekannten Hydrierungskatalysatoren für aromatische Nitroverbindungen verwendet. Gut geeignet sind die Metalle der 8. Nebengruppe des Periodensystems der Elemente, die beispielsweise auf Trägermaterialien wie Oxiden von Magnesium, Aluminium und/oder Silicium aufgebracht sein können. Vorzugsweise werden Raneyeisen, -kobalt und/oder -nickel, insbesondere Raneynickel, verwendet. Die Katalysatoren werden in feinverteiltem Zustand eingesetzt und liegen im Reaktionsgemisch feinteilig-suspendiert vor. Nicht nur bezüglich der Art sondern auch bezüglich der Menge des einzusetzenden Katalysators entspricht das erfindungsgemäße Verfahren den bislang bekanntgewordenen Verfahren des Standes der Technik. Die optimale Menge an Katalysator kann erforderlichenfalls durch einige orientierende Vorversuche ermittelt werden.

Die Durchführung des erfindungsgemäßen Verfahrens erfolgt in Anwesenheit von Tetrahydrofuran als wesentliches Reaktionsmedium. Das Tetrahydrofuran dient hierbei als Lösungsmittel für die Dinitroverbindungen und die resultierenden Diaminoverbindungen und zusammen mit diesen als Suspendiermedium für den eingesetzten Katalysator. Das Tetrahydrofuran wird bei der Durchführung des erfindungsgemäßen Verfahrens in einer Menge von 25 bis 80, vorzugsweise 35 bis 70 Gew.-%, bezogen auf das Gesamtgewicht des Reaktionsansatzes eingesetzt. Vorzugsweise werden die beim erfindungsgemäßen Verfahren einzusetzenden Dinitroverbindungen vorab in einer entsprechenden Menge an Tetrahydrofuran gelöst.

Das erfindungsgemäße Verfahren wird im allgemeinen bei einer Temperatur von 160 bis 250°C, vorzugsweise 180 bis 240°C und unter einem Druck von mehr als 10 bar, vorzugsweise 20 bis 150 bar durchgeführt. Der Wasserstoff wird hierbei, bezogen auf die Dinitroverbindungen, in hohem Überschuß eingesetzt und wird unter Aufrechterhaltung des genannten Drucks in den Reaktor eingeleitet.

Die Verteilung des suspendierten Kontakts im Reaktionsgemisch kann beispielsweise durch Umwälzung von Wasserstoff oder durch Rühren gewährleistet werden.

Das erfindungsgemäße Verfahren kann im Prinzip nach beliebigen Methoden und unter Verwendung von beliebigen Reaktionsapparaturen des Standes der Technik erfolgen, beispielsweise unter Verwendung einer Blasensäule gemäß EP-A 124 010 oder unter Verwendung von Rührwerksbehältern, die mit interne Filtern zur Rückhaltung des Katalysators kombiniert sein können (US-PS 3 431 085). Außerdem können selbstverständlich beliebige, bislang für Hydrierungsreaktionen unter erhöhtem Druck eingesetzte Autoklaven zur Durchführung des Verfahrens verwendet werden. Das Verfahren wird vorzugsweise kontinuierlich durchgeführt.

Die beim erfindungsgemäßen Verfahren anfallenden, den Katalysator im allgemeinen in suspendierter Form aufweisenden Reaktionsgemische, werden auf geeignete Weise, beispielsweise durch Filtrieren oder Sedimentieren vom suspendierten Katalysator befreit und an schließend destillativ aufgearbeitet. Bei dieser destillativen Aufarbeitung wird das Tetrahydrofuran zusammen mit einem Teil des bei der Hydrierung entstandenen und gegebenenfalls bereits zu Beginn der Reaktion eingesetzten Wassers als Kopfprodukt zurückgewonnen und kann ohne weitere Abtrennung des Wassers an den Prozeßanfang zurückgeführt werden. Dies bedeutet, daß das beim erfindungsgemäßen Verfahren eingesetzte Tetrahydrofuran bis zu 20, vorzugsweise bis zu 7 Gew.-%, bezogen auf Gemisch aus Tetrahydrofuran und Wasser, an Wasser enthalten kann.

Das erfindungsgemäße Verfahren gestattet die Herstellung von aromatischen Diaminoverbindungen in ausgezeichneten Ausbeuten, ohne nennenswerte Bildung von unerwünschten Nebenprodukten bei gleichzeitig vermindertem Katalysatorverbrauch, wobei die Umsetzung bei hohen Temperaturen erfolgen kann, so daß die Nutzung der Reaktionsenthalpie zur problemlosen Erzeugung von Dampf eines über 1 bar liegenden Überdrucks, insbesondere eines absoluten Drucks von 3 bis 20 bar genutzt werden kann.

In den nachfolgenden Beispielen beziehen sich alle Prozentangaben auf Gewichtsprozente.

## Beispiel

Die kontinuierliche Hydrierung von Dinitrotoluol in Tetrahydrofuran (THF) wird in einem Autoklaven mit 30 ml Flüssigvolumen durchgeführt. Der Autoklav ist mit einem Begasungsrührer und einer Sintermetallfritte ausgestattet, die den suspendierten Katalysator zurückhält. Der Druckreaktor ist außerdem mit einer Zuführungsleitung für Wasserstoff und einem Einleitungsrohr für die aromatische Dinitroverbindung, dessen unteres Ende direkt am Begasungsrührer endet, versehen. Die Temperatur im Reaktor wird durch einen äußeren Heiz-bzw. Kühlkreislauf geregelt.

40 ml eines Gemischs aus 62 Gew.-% THF (100 %ig), 16,4 Gew.-% Wasser und 21,6 Gew.-% eines Gemischs aus 80 % 2,4-Diaminotoluol und 20 % 2,6-Diaminotoluol, in dem 0,8 g eines Raney-Skelettkatalysators,bestehend zu 70% aus Nickel und zu 30% aus Eisen,suspendiert sind, werden in dem Reaktor vorgelegt. Dieses Gemisch entspricht der Zusammensetzung des bei der Durchführung des Verfahrens anfallenden Reaktionsgemischs. Anschließend wird der Reaktorinhalt unter einer Wasserstoffatmosphäre bei einem Druck von 80 bar auf 200°C erwärmt. Bei dieser Temperatur werden stündlich 150 l Wasserstoff und, aus einer Vorlage, 440 g einer Lösung von 145,2 g Dinitrotoluol (Gemisch aus 80 % 2,4-Dinitrotoluol und 20 % 2,6-Dinitrotoluol) in einem Gemisch aus 280 g THF (100 %ig) und 14,8 g Wasser (entsprechend der Zusammensetzung des bei der destillativen Aufarbeitung anfallenden, wäßrigen THF) mit einer Dosierpumpe in den Reaktor geleitet, wobei die Temperatur auf 220°C steigt. Bei dieser Temperatur und einem Druck von 80 bar wird bis zur Erschöpfung des Katalysators hydriert. Das Reaktionsgemisch (Diaminotoluol, Wasser und THF) strömt über die Sintermetallfritte kontinuierlich in eine Vorlage und wird von dieser einer destillativen Aufarbeitungsstufe zugeleitet. Der überschüssige Wasserstoff und gasförmige Nebenkomponenten werden über ein Druckhalteventil ausgeschleust.

Die durchschnittliche Ausbeute an 2,4-und 2,6-Diaminotoluol (TDA) liegt bei 98,8 %, bezogen auf eingesetzte Dinitroverbindungen. Der spezifische Katalysatorverbrauch liegt bei 0,024 kg pro 100 kg TDA.

Vergleichsbeispiel 1

Das Beispiel wird wiederholt, mit dem einzigen Unterschied, daß das Tetrahydrofuran durch die gleiche Menge Isopropanol ersetzt und die Reaktionstemperatur bei 200°C gehalten wird. Bei gleichem Hydrierdruck resultiert eine Ausbeute an TDA von lediglich 98,3% bei einem spezifischen Katalysatorverbrauch von 0,035 kg pro 100 kg TDA.

Vergleichsbeispiel 2

Das Beispiel wird wiederholt, mit dem einzigen Unterschied, daß überhaupt kein organisches Lösungsmittel eingesetzt wird. Bei gleichem Hydrierdruck resultiert eine Ausbeute von 97,1 % TDA bei einem spezifischen Katalysatorverbrauch von 0,09 kg pro 100 kg TDA. Bei beiden Vergleichsversuchen sind nach Beendigung der Umsetzung die Innenwände der Apparaturen mit einem zähen Harz belegt.

**Ansprüche**

1. Verfahren zur Herstellung von aromatischen Diaminen durch katalytische Hydrierung der ihnen entsprechenden aromatischen Dinitroverbindungen in Gegenwart eines organischen Lösungsmittels, dadurch gekennzeichnet, daß man als organisches Lösungsmittel Tetrahydrofuran verwendet.

2. Verfahren gemäß Anspruch 1, dadurch gekennzeichnet, daß man die Hydrierung bei einer Temperatur von 160 bis 250°C und unter einem Druck von mehr als 10 bar durchführt.

3. Verfahren gemäß Anspruch 1 und 2, dadurch gekennzeichnet, daß man das Tetrahydrofuran in einer Menge von 25 bid 80 Gew.-%, bezogen auf das gesamte Reaktionsgemisch, verwendet.

4. Verfahren gemäß Anspruch 1 bis 3, dadurch gekennzeichnet, daß man die Reaktionsenthalpie zur Erzeugung von Dampf mit einem über 1 bar liegenden Überdruck nutzt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE-B-1 085 165 (B.A.S.F.) <br> * Beispiel 3 * | 1-4 | C 07 C 85/11 |
| | --- | | |
| X | FR-A-1 385 334 (MOBAY CHEMICAL CO.) <br> * Seite 3, Zeilen 33-45; Patentansprüche * | 1-4 | |
| | --- | | |
| X | FR-A-2 565 970 (MITSUI TOATSU CHEMICALS INC.) <br> * Beispiel 2 * | 1-4 | |
| | --- | | |
| D,X | EP-A-0 124 010 (BAYER AG) <br> * Patentansprüche * | 1,4 | |
| | ----- | | **RECHERCHIERTE SACHGEBIETE (Int. Cl.4)** |
| | | | C 07 C 85/00 |

Der vorliegende Recherchenbericht wurde für alle Patentansprüche erstellt.

| Recherchenort | Abschlußdatum der Recherche | Prüfer |
|---|---|---|
| DEN HAAG | 17-07-1987 | PAUWELS G.R.A. |

KATEGORIE DER GENANNTEN DOKUMENTE
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82